(19) Europäisches Patentamt
European Patent Office
Office européen des brevets

(11) **EP 3 698 777 A1**

(12) # EUROPEAN PATENT APPLICATION

(43) Date of publication:
26.08.2020 Bulletin 2020/35

(21) Application number: 20158390.3

(22) Date of filing: 20.02.2020

(51) Int Cl.:
*A61K 9/48* *(2006.01)*    *A61K 31/202* *(2006.01)*
*A61K 31/351* *(2006.01)*    *A61K 36/744* *(2006.01)*
*A61K 45/06* *(2006.01)*

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
KH MA MD TN

(30) Priority: 20.02.2019 IT 201900002457

(71) Applicant: NEURAXPHARM ITALY S.P.A.
63100 Ascoli Piceno (AP) (IT)

(72) Inventors:
• MARCHETTI, Marco
63074 SAN BENEDETTO DEL TRONTO (IT)
• IPAVEC, Valeria
00148 ROMA (IT)
• MAGAGNINI, Mattia
60127 Castelfidardo (IT)

(74) Representative: Coppo, Alessandro et al
Notarbartolo & Gervasi S.p.A.
Viale Achille Papa, 30
20149 Milano (IT)

(54) **COMPOSITION FOR PREVENTING AND TREATING DEPRESSIVE AND COGNITIVE SYMPTOMS**

(57) In one aspect, the present invention concerns a kit comprising a composition containing an omega-3 fatty acid and crocin and a physiologically acceptable carrier and a composition containing N-acetylcysteine and crocin or a saffron extract containing crocin and a physiologically acceptable carrier. The kit finds application in the prevention or treatment of a depressive disorder or a symptom thereof and a symptom of cognitive impairment.

**Description**

FIELD OF THE INVENTION

The present invention relates to a composition for preventing or treating depressive and cognitive symptoms.
The present invention originates in the field of nutraceuticals, pharmaceuticals and food supplements.
In particular, the present invention concerns a preparation for oral administration suitable for preventing or treating symptoms of depression and cognitive disorders.

PRIOR ART

Several neurobiological mechanisms and factors underlie the pathophysiology of depression. It is currently believed that various factors, such as impairment of the monoaminergic system, neuroendocrine changes, reduced neurogenesis, redox reactions underlying oxidative, and alterations in cytokines consistent with chronic inflammation, contribute to determining the depressive disorder.
Inflammation and activation of the hypothalamic-pituitary-adrenal axis are physiological components of response to stress. It has been seen that, in the presence of prolonged stress, endocrine and immune systems are chronically activated and consequently a high activation of peripheral macrophages and central microglia is observed together with hypercortisolemia. Under these conditions, neuronal networks are damaged and their functionality is impaired.
Furthermore, under stress conditions, proinflammatory cytokines not only activate the hypothalamic-pituitary-adrenal (HPA) axis, leading to an increase in the synthesis of cortisol, but also activate the tryptophan-kynurenine pathway.
In this situation, the synthesis of quinolinic acid, a neurotoxic NMDA agonist, and 3-hydroxykynurenine is increased, with consequent increase in oxidative stress. This circumstance contributes to neurodegeneration, a condition that accompanies some neuropsychiatric diseases including depression.
It has also been observed that the inflammatory response affects the neurotransmitter system and neurocircuits that regulate the behavior and are involved in some diseases, such as depression and anxious-depressive disorder, that are often accompanied by anhedonia, i.e. reduction of motivation, and avoidance syndrome and excessive alarmism.
Under these conditions, at the molecular level pro-inflammatory cytokines frequently reduce the availability of monoamines, such as 5-HT, NA, DA, increasing the expression of presynaptic transporters and reducing their synthesis. In addition, both microglia activation and oxidative stress, can lead to excess glutamate, excitotoxicity, and decrease in neurotrophin levels (BDNF).
Inflammation, with its effects on neurotrophins, can also adversely affect neuronal plasticity, learning skills and memory.
Finally, it has recently been observed that the brain, having a high oxygen consumption and being rich in lipids, is highly susceptible to oxidative stress, a condition that occurs when, following a high production of free radicals, there is an inadequate response of the body anti-oxidant system. Consequently, brain damage induced by oxidative stress can have a strong negative impact on central nervous system (CNS) functions.
It has also been observed that neuropsychiatric diseases are accompanied by low antioxidants levels and an increase in nitrogen (RNS) and oxygen (ROS) reactive species, that activate the nuclear factor (Nrf2) thus increasing the expression of endogenous antioxidants.
In turn, ROSs increase the consumption of antioxidant defenses. The consequences of oxidative and nitrosative stress (O&NS) pathways activation determine the formation of oxidation-specific epitope (OSE) and nitrosative specific epitopes (NSE), that can be immunogenic and therefore can cause autoimmune responses, oxidative damage, nitrosative damage, and hypernitrosylation to various cellular components.
The oxidative and nitrosative stress pathways determine consequences in the human body and cause a wide variety of cellular dysfunctions.
In addition, there are a number of mitochondrial alterations that were observed both in psychiatric patients and animal depression models, changes that affect mitochondrial DNA, increase membrane permeability, and cause an increase in the formation of reactive oxygen species (ROS).
These alterations determine pro-inflammatory activity, increase of apoptosis and reduction of both synaptic plasticity and neuronal differentiation.
The different etiology of cognitive disorders means that about 5% of the population over the age of 65 and about 30% of the population over the age of 85 are affected.
Currently, mild forms of cognitive impairment are treated by supplementing the diet with nutritional products containing vitamins, substances with antioxidant activity such as flavonoids, selenium, resveratrol, or substances with a phospholipid matrix such as phosphatidylserine, phosphatidylcholine or phosphatidylinositol. However, nutraceutical products currently on the market to manage cognitive and depressive symptoms provide an inadequate, if not entirely unsatisfactory, therapeutic response.

As a result of this situation, a considerable number of patients, even if suffering from mild symptoms, use antidepressant drugs such as sertraline, or monoamine oxidase, or medicines for the treatment of more sever forms of cognitive impairment such as acetylcholinesterase inhibitors, such as donepezil, rivastigmine and galantamine.

However, it is found daily how the administration of these drugs is accompanied by side effects of certain relevance.

Therefore, there is currently a need to have preparations, such as nutraceuticals or food supplements, whose intake contributes to managing the most common depression symptoms and the related cognitive symptoms, whose use is substantially free of side effects, even in the case of long-term treatments.

Therefore, a general object of the present invention is to provide a preparation, possibly in the form of a kit in parts, suitable for improving the clinical condition and/or symptoms linked to mild or moderate depression disorders and cognitive disorders.

Another object of the present invention is to provide a composition or kit for preventing or treating cognitive disorders, whose administration allows treatment and/or long-term management of the main depressive and cognitive symptoms.

## SUMMARY OF THE INVENTION

According to a first aspect of the present invention, the inventors found that by administering a combination of three selected substances such as N-acetylcysteine, omega-3 fatty acids and crocin, a synergistic antidepressant action is obtained which is accompanied by an improvement of cognitive abilities and a slow-down in the progression of cognitive functions decline in the subject treated.

It was found that the simultaneous, separate or subsequent administration of N-acetylcysteine, omega-3 fatty acids and crocin affect some pathways that are implicated in mood regulation and play a role in depressive disorders and cognitive impairment disorders related to a depression condition.

According to a first aspect, a kit in parts is therefore supplied according to what is claimed in the appended claim 1.

Further embodiments of the kit of the invention are defined in claims 2-6 appended herein.

According to a general aspect, the present invention therefore provides a composition or kit for the prevention and/or treatment of a depressive disorder or one or more symptoms of depression and/or related cognitive symptoms in a human being.

Typically, in the composition of the invention the biologically active components contributing to the achievement of the antidepressant activity are provided in a nutraceutically, pharmaceutically or dietetic effective amount.

The composition or kit of the invention find application in the prevention or treatment of depression symptoms and/or cognitive impairment regardless of their origin. The composition or kit described herein also find application as adjuvants against the symptoms associated with these disorders.

According to some embodiments, the composition of the invention is a pharmaceutical composition, a nutraceutical, a food supplement that can be introduced into the dietary regimen of an individual suffering from a symptom attributable to a depressive disorder and/or a cognitive disorder.

According to some aspects, the invention provides a kit comprising

a) a composition, typically in the form of capsules, comprising an omega-3 fatty acid and crocin and a physiologically acceptable carrier

b) a composition comprising N-acetylcysteine and crocin and a physiologically acceptable carrier

as a combined preparation for simultaneous, separate or subsequent administration particularly in the prevention or treatment of a symptom linked to a depressive disorder or a cognitive disorder typically linked to a depressive disorder.

According to preferred embodiments, a sequential administration of components a) and b) of the kit of the invention is provided comprising an administration step of composition a) and a second administration step of composition b), wherein the two steps may be carried out simultaneously, separately or in sequence such as a) and then b) or b) and then a).

In some embodiments, crocin is contained in a saffron extract in which typically safranal and optionally other biologically active components are present.

According to another aspect, a kit as previously described is provided for use in the prevention or treatment of depression and/or neurodegenerative diseases and/or cognitive disorders.

According to a further aspect, the present invention concerns a method for the prevention or treatment of depression and cognitive impairment symptoms, comprising the administration to an individual of a kit comprising capsules comprising an omega-3 fatty acid and crocin and a physiologically acceptable carrier, and tablets comprising N-acetylcysteine and crocin and a physiologically acceptable carrier.

BRIEF DESCRIPTION OF THE FIGURES

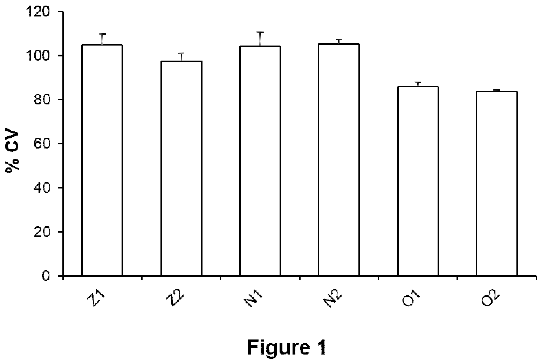

Figure 1 shows a graph of the MTT analysis on human microglial HMC3 cells in order to detect cell viability after treatment with the substances of the invention (Z1: saffron extract, 3 $\mu$g/mL; Z2: saffron extract, 1 $\mu$g/mL; N1: NAC, 600 $\mu$M; N2: NAC, 200 $\mu$M; O1: fish oil, 28.5 $\mu$g/mL; O2: fish oil, 8.5 $\mu$g/mL). Cell viability values (% CV) are expressed as a percentage of untreated control cells.

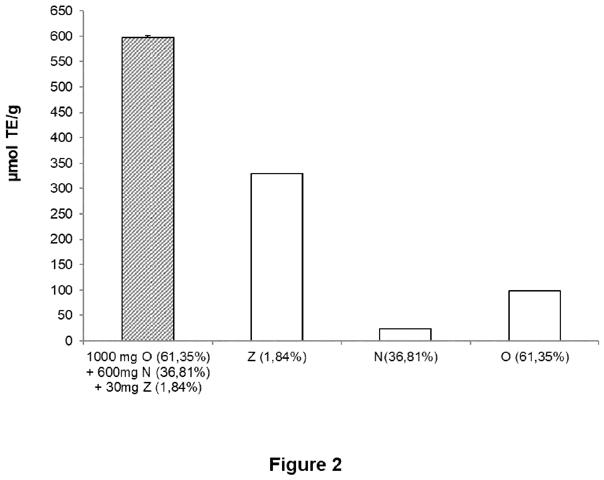

Figure 2 shows a graph of the ORAC test carried out with substances of the invention (Z: dry saffron extract; N: NAC e O: fish oil) alone or in combination. The antioxidant activity of both a combination of 1000 mg of fish oil + 600 mg of NAC + 30 mg of dry saffron extract (striped bar) and the single ingredients (white bars) taking into account the percentage composition of the combination, was determined and expressed as Trolox equivalent micromol/sample gram (i.e. TE $\mu$mol/g).

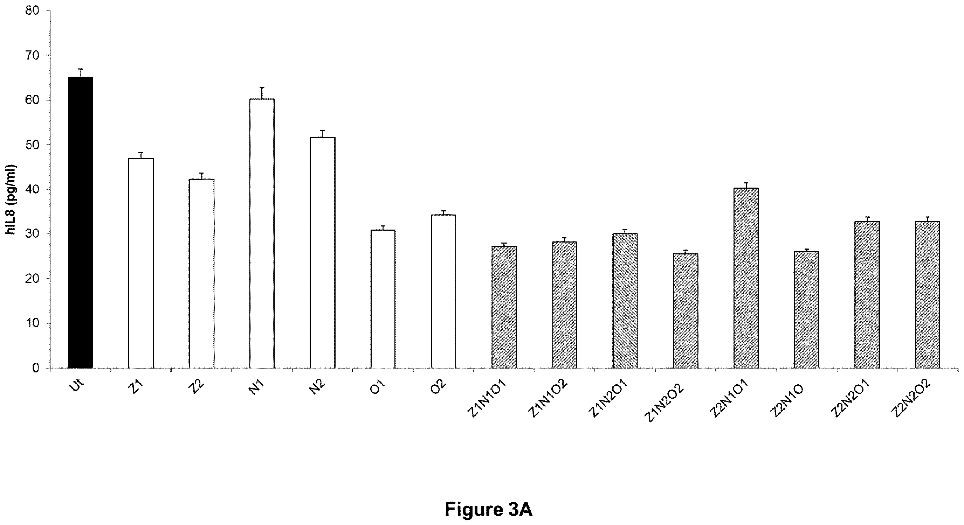

Figure 3A shows a graph of hIL8 level (in pg/mL) of untreated cells (ut; black bar) and cells treated with different concentrations of the substances of the invention (Z1: saffron , 3 $\mu$g/mL; Z2: saffron extract, 1 $\mu$g/mL; N1: NAC, 600 $\mu$M; N2: NAC, 200 $\mu$M; O1: fish oil, 28.5 $\mu$g/mL; O2: fish oil, 8.5 $\mu$g/mL) alone (white bars) or in combination (striped bars) at basal conditions, i.e. without any inflammatory stimulus.

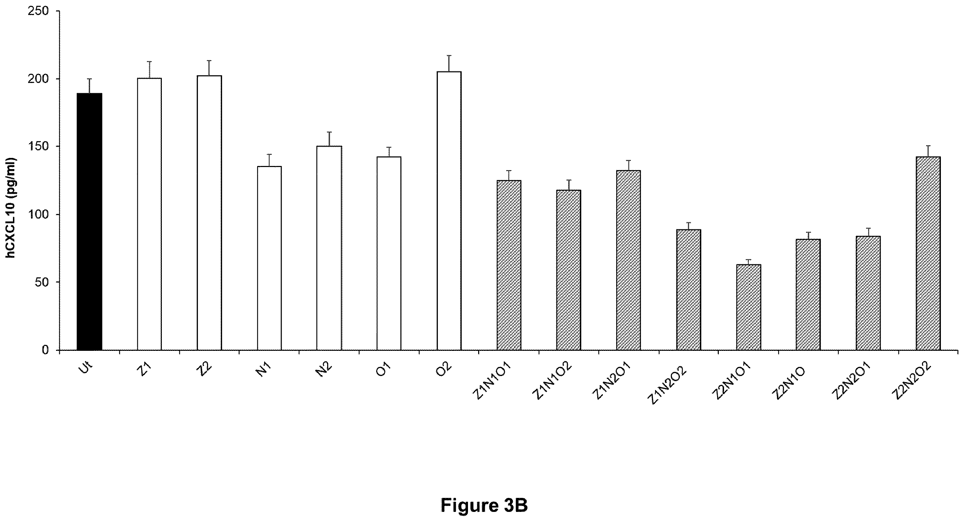

Figure 3B shows a graph of hCXCL10 level (in pg/mL) of untreated cells (ut; black bar) and cells treated with different concentrations of the substances of the invention (Z1: saffron extract, 3 $\mu$g/mL; Z2: saffron extract, 1 $\mu$g/mL; N1: NAC, 600 $\mu$M; N2: NAC, 200 $\mu$M; O1: fish oil, 28.5 $\mu$g/mL; O2: fish oil, 8.5 $\mu$g/mL) alone (white bars) or in combination (striped bars) at basal conditions, i.e. without any inflammatory stimulus.

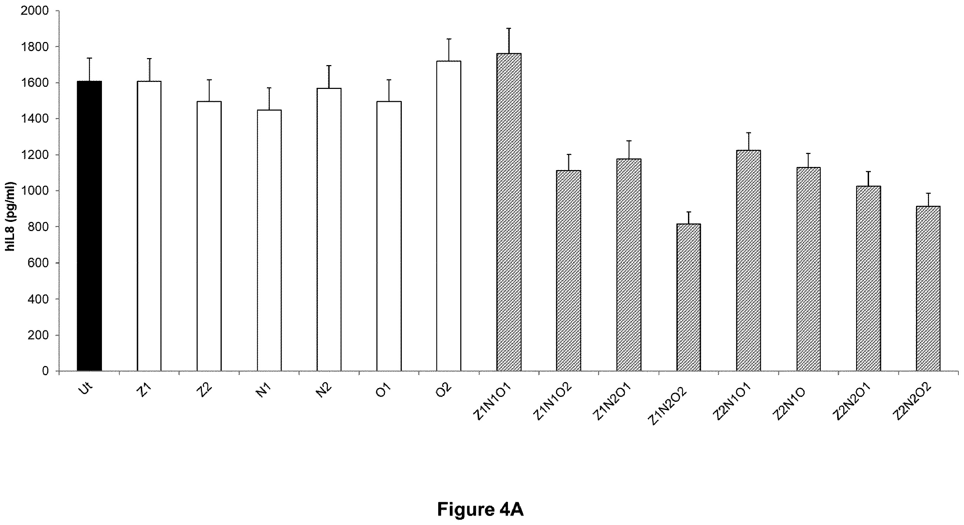

Figure 4A shows a graph of hIL8 level (in pg/mL) of untreated cells (ut; black bar) and cells treated with different concentrations of the substances of the invention (Z1: saffron extract, 3 $\mu$g/mL; Z2: saffron extract, 1 $\mu$g/mL; N1: NAC, 600 $\mu$M; N2: NAC, 200 $\mu$M; O1: fish oil, 28.5 $\mu$g/mL; O2: fish oil, 8.5 $\mu$g/mL) alone (white bars) or in combination (striped bars), that were stimulated with TNF$\alpha$.

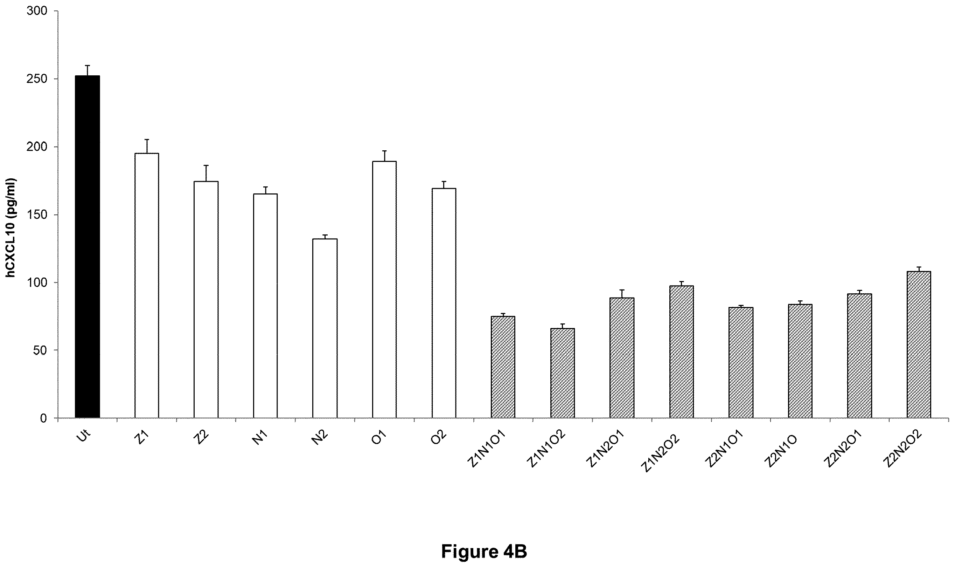

Figure 4B shows a graph of hCXCL10 level (in pg/mL) of untreated cells (ut; black bar) and cells treated with different concentrations of the substances of the invention (Z1: saffron extract, 3 $\mu$g/mL; Z2: saffron , 1 $\mu$g/mL; N1: NAC, 600 $\mu$M; N2: , 200 $\mu$M; O1: fish oil, 28.5 $\mu$g/mL; O2: fish oil, 8.5 $\mu$g mL) alone (white bars) or in combination (striped bars), that were stimulated with TNF$\alpha$.

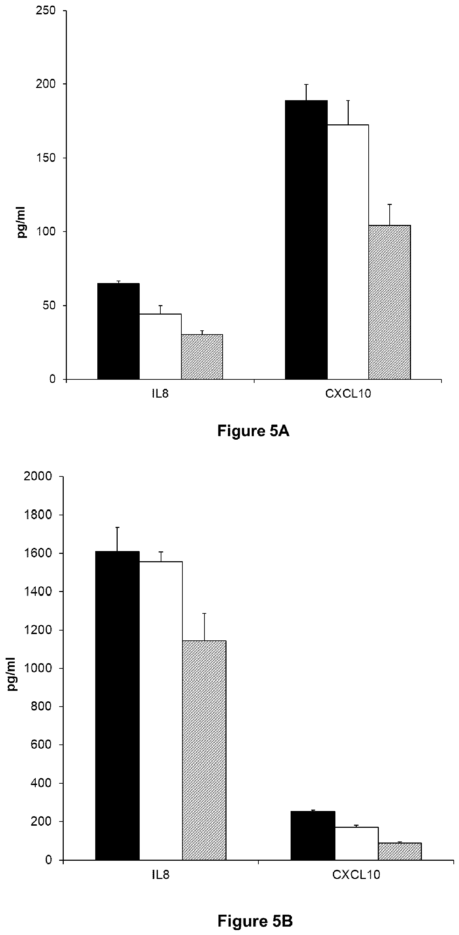

Figure 5A shows a graph of the mean levels of hIL8 and hCXCL10 at the basal conditions shown in Figures 3A (hIL8 levels) and 4A (hCXCL10 levels). The black bars correspond to cytokines levels in untreated cells (ut); the white bars correspond to the mean levels of cytokine in cells treated with the single ingredients, and the striped bars correspond to the mean levels of cytokine in cells treated with the combinations of ingredients. The mean level of cytokines is expressed in pg/mL.

Figure 5B shows a graph of the mean levels of hIL8 and hCXCL10 at the time of stimulation with TNF$\alpha$ shown in Figures 3B (hIL8 levels) and 4B (hCXCL10 levels). The black bars correspond to the levels of cytokines in untreated cells (ut); the white bars correspond to the mean levels of cytokines in cells treated with the single ingredients, and the striped bars correspond to the mean levels of cytokines in cells treated with the combinations of ingredients. The mean level of cytokines is expressed in pg/mL.

DETAILED DESCRIPTION OF THE INVENTION

According to some aspects of the invention, the inventors found that the simultaneous, separate or subsequent administration of crocin, or a saffron extract containing crocin, with N-acetylcysteine and/or an omega-3 fatty acid reduce one or more symptoms of depression and possibly related symptoms of cognitive decline, by acting on metabolic pathways such as inflammation and oxidative stress pathways considered to be only marginally implicated in the origin of depression and cognitive disorders.

The biological action exerted by the synergistic composition of the invention is surprising since it is only partially related with an action exerted by neurotransmitters physiologically present in the human body.

The action of the composition/kit of the invention is attributable to a multi-target action exerted by the combination of the biologically active components N-acetylcysteine, omega-3 fatty acid, and crocin on the central, inflammatory and redox transmission cell pathways.

In particular, it was found that taking crocin in combination with N-acetylcysteine and/or an omega-3 fatty acid reduces neuroinflammation and cellular oxidative stress, and contributes to maintaining physiological neurogenesis in the treated human organism, in the substantial absence of most common side effects experienced with use of antidepressant or drugs against brain impairment.

According to one aspect, the present invention therefore relates to a kit comprising

a) crocin and an omega-3 fatty acid and a physiologically acceptable carrier and

b) crocin and N-acetylcysteine and a physiologically acceptable carrier.

The kit of the invention is for simultaneous, separate or subsequent use, in particular in the prevention or treatment of a symptom linked to a depressive disorder or to a cognitive disorder typically linked to a depressive disorder.

A component of the composition or kit of the invention is N-acetylcysteine.

N-acetylcysteine, also called NAC, is an N-acetylated derivative of the sulfur amino acid cysteine.

Diet supplementation with N-acetylcysteine reduces the levels of 8-hydroxy-deoxyguanosine, an indicator of oxidative damage, and the frequency of DNA deletions. In addition, N-acetylcysteine is a precursor of Glutathione, a substance that reduces the formation of free radicals and reactive oxygen compounds (ROS). N-acetylcysteine activates glutamatergic neurotransmission. In particular, it modulates the activity of the cystine/glutamate exchanger and indirectly activates the mGlu2/3 receptor by regulating the extracellular levels of glutamate. The regulation of glutamate levels contributes to the maintenance of synaptic plasticity and the reduction of neuronal damage, as well as modulation of dopaminergic and serotonergic transmission.

According to some embodiments, the NAC present in the composition of the invention has a titer higher than 55%, higher than 95%, higher than 98%.

In preferred embodiments, the NAC used in the composition is highly pure and is almost entirely free of pollutants such as allergens, heavy metals, additives and preservatives, for example each of these contaminants may be present in an amount equal to or lower than 0.001%.

The NAC used in the formulation of the composition of the invention can be of any origin and can be obtained through conventional production processes.

According to some embodiments, the composition or kit of the invention contains N-acetylcysteine in an amount of 50 to 5000 mg, 100 to 2000 mg, 250 to 750 mg.

According to some embodiments, the composition or kit of the invention contains N-acetylcysteine in an amount of 0.1 to 95% by weight, 1 to 90% by weight, 10 to 85%, 30 to 80 by weight.

In addition to having an activity on neurotransmission, it has been observed that NAC also exerts an action on cells oxidative stress and neuroinflammation. Specifically, NAC acts as an antioxidant and as a precursor of glutathione, it also reduces the activation of the NF-kB transcription factor, the expression of pro-inflammatory genes and the related synthesis of cytokines.

The Applicant has surprisingly observed that by combining NAC with an omega-3 acid and crocin, a synergistic effect on the prevention and treatment of depression, particularly mild or moderate disorders, and related cognitive disorders, is obtained.

Omega-3 acids used within the scope of the invention fall into the category of polyunsaturated fatty acids, called PUFAs.

Within the scope of the invention it is possible to use any omega-3 acid such as, for example, hexadecatrienoic acid (HTA), octadecatrienoic acid (ALA), octadecatetraenoic acid (SDA), eicosatrienoic acid (ETE), eicosatetraenoic acid (ETA), acid eicosapentaenoic acid (EPA), eneicosapentaenoic acid (HPA), docosapentaenoic acid (DPA), docosahexaenoic acid (DHA), tetracosapentaenoic acid, tetracosahexanoic acid. Among these omega-3 acids, EPA and DHA are preferred.

Omega-3 PUFAs are essential components of cell membranes and represent regulatory factors in neurotransmission, neurogenesis, response to stress, inflammation and emotional states. They play an important role in the development, functioning and aging of central nervous system.

Omega-3 PUFAs contribute to the maintenance of mental health, prevention and improvement of various psychopathological conditions.

Omega-3s are involved in many mechanisms. The anti-inflammatory activity is partially due to an antagonistic action on metabolites of omega-6 fatty acids, such as arachidonic acid derivatives, known to have pro-inflammatory activity. Furthermore, Omega-3 fatty acids and particularly DHA and EPA inhibit the release of proinflammatory cytokines, such as INF-y, TNF-$\alpha$, IL-1, IL-2 and IL-6, by directly acting on the transcription factor NF-kB.

Furthermore, Omega-3 PUFAs are involved in the regulation of response to stress by influencing the activity of the HPA axis ("hypothalamic-pituitary-adrenal axis").

Omega-3s, mainly DHA, also regulate BDNF levels by positively influencing synaptic plasticity, memory and learning abilities.

Furthermore, associations between monoaminergic dysfunction in stress-related diseases and lower diet levels of Omega-3 PUFAs have been observed, and the ability of Omega-3 PUFAs to regulate the glutamatergic system function, that can undergo dysregulation during aging, has been observed.

Finally, Omega-3 PUFAs decrease A$\beta$ levels and have antioxidant and antiapoptotic effects, thus protecting neurons and contributing to the maintenance of learning-memory ability. According to some aspects of the present invention, these properties are enhanced when Omega-3s are administered together with NAC and crocin.

In some embodiments, the Omega-3 acids of the composition or kit of the invention are contained in fish oil.

In certain embodiments, the composition or a component of the kit of the invention contain fish oil containing an Omega-3 acid, in particular EPA and/or DHA, in an amount of 10 to 3000 mg, 50 to 2000 mg, 100 to 1500 mg.

In some embodiments, the composition or a component of the kit typically comprise EPA in an amount of 10 to 3000mg, 50 to 1000 mg, preferably of 100 to 800 mg, for example 200 to 600 mg.

In some embodiments, the composition or a component of the kit typically comprise DHA in an amount of 5 to 1500 mg, 30 to 1000 mg, preferably of 50 to 400 mg, for example 100 to 300 mg.

In certain embodiments, the composition or a component of the kit of the invention contains an omega-3 acid, in particular EPA and/or DHA, in an amount of 0.1 to 40% by weight, 0.5 to 20% by weight, 2 to 15% by weight.

Another component of the composition or kit of the invention is crocin. A suitable source of crocin is represented by saffron.

In some embodiments, the composition or a component of the kit of the invention includes a saffron extract, preferably dry saffron extract, in an amount of 5 to 100 mg, preferably 5 to 60 mg, more preferably 10 to 50 mg.

The composition or component of the kit of the invention may contain crocin as such or saffron containing a biologically active amount of crocin.

Chemically, crocin is the diester formed from the disaccharide gentiobiose and the dicarboxylic acid crocetin.

It has been observed that, having a 5-HT2c antagonistic action, crocin influences the serotonergic mechanisms, contributing to the antidepressant activity of the composition and helping to reduce related cognitive disorders.

In addition, crocin increases the levels of CREB (cAMP response element-binding protein) and BDNF in a dose-dependent manner and, at higher levels, increases the concentration of VGF (a neuropeptide) in the hippocampus.

In some embodiments, the composition or a component of the kit of the invention comprise crocin in an amount of 100 to 5000 $\mu$g, 100 to 2000 $\mu$g, preferably of 500 to 1500 $\mu$g.

According to some embodiments, crocin is contained in saffron, typically in the form of a dry extract. According to these embodiments, the composition contains saffron. Typically, in addition to crocin, saffron contains other biologically active components including picrocrocin, safranal, crocetin, a crocin hydrolysis product, whose presence in the composition can further contribute to biological activity and use as claimed.

It has been observed that saffron and constituents thereof, crocin, crocetin and safranal, are powerful cellular antioxidants and the combination of crocin and safranal increases these effects synergistically. Saffron also has an anti-inflammatory effect, by exerting a direct activity towards COX-1 and COX-2.

In addition, crocin and crocetin reduce the production of TNF-$\alpha$, IL-1 $\beta$ from lipopolysaccharide-stimulated microglial cells, and the activation of factor NF-$\kappa$B.

In the embodiment wherein the crocin of the composition of the invention is contained in a saffron extract, said extract can further contain safranal, typically in an amount of 30 to 2000 $\mu$g, 60 to 1000 $\mu$g, preferably of 100 to 600 micrograms.

The Applicant observed that the separate or contextual administration of N-acetylcysteine, Omega-3 and saffron reduce the levels of inflammation mediators and oxidative stress, by acting at different levels of the signaling, transcription and cellular metabolic pathways. These biological actions contribute to the synergistic antidepressant action of the composition described herein.

In accordance to that, in some embodiments the kit of the invention includes:

a) a composition comprising fish oil in an amount of 10 to 3000 mg, a dry extract of saffron in an amount of 5 to 100 mg and a physiologically acceptable carrier, and
b) a composition comprising N-acetylcysteine in an amount of 50 to 5000 mg, a dry extract of saffron in an amount of 5 to 100 mg and a physiologically acceptable carrier.

In accordance to that, in some embodiments, the kit of the invention comprises:

a) a composition comprising fish oil in an amount of 100 to 1500 mg, a dry extract of saffron in an amount of 10 to 50 mg and a physiologically acceptable carrier, and
b) a composition comprising N-acetylcysteine in an amount of 250 to 750 mg, a dry extract of saffron in an amount of 10 to 50 mg and a physiologically acceptable carrier.

In accordance to that, in some embodiments, the kit of the invention comprises:

a) a composition comprising an omega-3 fatty acid, in particular EPA in an amount of 10 to 3000 mg and/or DHA in an amount of 5 to 1500 mg, crocin in an amount of 100 to 5000 $\mu$g and a physiologically acceptable carrier and
b) a composition comprising N-acetylcysteine in an amount of 50 to 5000 mg, crocin in an amount of 100 to 5000 $\mu$g, and a physiologically acceptable carrier.

In accordance to that, in some embodiments, the kit of the invention comprises:

a) a composition comprising an omega-3 fatty acid, in particular EPA in an amount of 200 to 600 mg and/or DHA in an amount of 100 to 300 mg, crocin in an amount of 500 to 1000 $\mu$g and a physiologically acceptable carrier and
b) a composition comprising N-acetylcysteine in an amount of 250 to 750 mg, crocin in an amount of 500 to 1000 $\mu$g, and a physiologically acceptable carrier.

In the present application, the term "synergism or synergistic activity" means an activity that is greater than the sum of the activities of each single active substance. In particular, synergism occurs when at least two active substances or active ingredients interact in a way that enhances or increases one or more effects thereof. Therefore, two substances or active ingredients that clearly produce similar effects will sometimes produce exaggerated or reduced effects when used simultaneously and a quantitative assessment is necessary to distinguish these cases from a simple additive action (Tallarida RJ. Drug Synergism: its detection and applications. J PharmacolExpTher. 2001 Sep:298(3):865-72).

In some embodiments, the composition or a component of the kit of the invention may comprise one or more additional substances or active ingredients.

In some embodiments, the composition or kit of the invention further comprises one or more vitamins, for example B vitamins, niacin, vitamin A, vitamin C, vitamin PP, the B vitamins being preferred.

According to some embodiments, the composition of the invention further comprises one or more micronutrients and/or minerals such as salts of Mg, K, Na, Zn, Fe, Cr, Se, Mn and others.

As used herein, the term "carrier" indicates a medium, excipient, diluent with which the association of therapeutic or active ingredients is administered.

Any carrier and/or excipient suitable for the form of preparation desired for administration to human beings is contemplated for use with the compounds described in the present invention.

For the purposes of this application, the term "physiologically acceptable" is meant to indicate edible substances that are approved by the health authorities for use in pharmaceutical, phytotherapy, nutritional or food applications.

A physiologically acceptable carrier can be a pharmaceutically acceptable carrier and/or an edible carrier.

Within the scope of the present application, the term combination means that one or more active ingredients are added or mixed with one or other ingredients.

Therefore, the term combination or association should not be understood in the sense that the active ingredients are linked to one another by formation of chemical or other bonds.

The compositions or kit of the present invention comprise any composition made by administering the association of active ingredients of the present invention and a physiologically or pharmaceutically acceptable carrier. Such compositions are suitable for food, nutritional, pharmaceutical or dietary use in mammals, particularly in human beings.

The composition or a component of the kit of the invention can be in a wide variety of forms of preparation, depending on the desired route of administration. The composition or a component of the kit of the invention can be in a solid form.

When the composition or a component of the kit of the invention is in a solid form, it can be in the form of a tablet, capsule, powder, granules.

When the composition or a component of the kit is in a liquid form, it can be in the form of a suspension, emulsion, solution. In these cases, the carrier is a liquid and can be selected, for example, from water, glycols, oils, alcohol and mixtures thereof.

Typically, the composition or a component of the kit of the invention are in a solid form, for example in the form of a tablet or capsule.

Preparations in a solid form may comprise one or more carriers, such as starches, sugars, microcrystalline cellulose, and optionally diluents, granulation agents, lubricants, binders, disintegrating agents.

Typically, the tablets, pills, capsules, granules of a component of the kit may also contain a binder such as tragacanth gum, acacia, corn starch or gelatin; excipients such as dicalcium phosphate; a disintegrating agent such as corn starch, potato starch, alginic acid; a lubricant such as magnesium stearate; a sweetening agent such as sucrose, lactose or saccharin. If desired, the tablets may be coated, for example with a gastro-resistant film, using traditional techniques.

When the pharmaceutical unit form of the kit is a capsule, it can be a soft gel capsule. The capsule may contain a liquid carrier such as an oil, typically fish oil as previously described.

According to certain embodiments, the composition or each of the components of the kit of the invention contain a cellulose-based excipient which includes i) organic esters of cellulose, for example selected from cellulose acetate, cellulose propionate, cellulose triacetate, cellulose acetate propionate, cellulose acetate butyrate, ii) inorganic esters of cellulose, for example selected from nitrocellulose, cellulose sulphate, iii) ethers of cellulose selected from a) alkyl ethers of cellulose, for example selected from methyl cellulose, ethyl cellulose, ethyl methyl cellulose; b) hydroxyalkyl of ethers cellulose, for example selected from hydroxyethyl cellulose, hydroxypropyl cellulose, hydroxypropyl cellulose, hydroxyethyl cellulose, hydroxypropyl methyl cellulose, ethylhydroxyethyl cellulose; c) carboxyalkyl cellulose, for example carboxymethyl cellulose, salts and mixtures thereof. In certain embodiments, the cellulose-based excipients are crosslinked with physiologically acceptable crosslinking agents.

The composition or each component of the kit may also be present flavouring agents, preservatives, colouring agents, and the like.

In certain embodiments, the composition or component of the kit of the invention is in a solid form of a tablet. According to preferred embodiments, a component of the kit of the invention is in the form of a gastro-resistant tablet.

According to certain embodiments the tablet is in a form for modulated or controlled release of the active ingredients. By way of example, the composition or component of the kit may be a delayed release, fast release or slow-fast release formulation. Typically, the formulations with modulated or controlled release contain one or more cellulose-based excipients, in particular of the type previously described.

In certain embodiments, the composition or a component of the kit of the invention comprises as an excipient a hydrogenated fatty acid, preferably having a 3 to 20 carbon atoms chain, 14 to 18 carbon atoms. A typical example of a suitable hydrogenated fatty acid is hydrogenated palm oil.

In some embodiments, the active ingredients contained in the composition of the present invention can be combined or mixed as active ingredients in an intimate mixture with a suitable edible carrier and/or an excipient according to pharmaceutical and conventional food or nutrition industry technologies.

The compositions for pharmaceutical or nutritional use can be suitably presented in a single pharmaceutical form and prepared by any of the methods well known in the pharmaceutical or food art.

The amount of active compound in the composition or in a component of the kit of the invention is such as to obtain a dosage that prevents or is therapeutically effective on a depression disorder and associated cognitive disorder.

In some embodiments, the composition or a component of the kit of the invention further comprises one or more additional components such as additives, fillers, stabilizers, emulsifiers, texturing agents, film coating agents, plasticizers, wetting agents, and thickeners.

Various other materials may be present as coatings or to modify the physical form of the pharmaceutical unit. For example, the tablets can be coated with shellac, sugar or both. To prevent disintegration during transit through the upper part of the gastrointestinal tract, the composition may be a formulation with a gastro-resistant or enteric coating.

In some embodiments, in the composition or in a component of the kit of the present invention, the active ingredients are usually formulated in dosage units. The dosage unit can contain 0.1 to 1,000 mg of each active ingredient per dosage unit for daily administration.

In some embodiments, the formulation or a component of the kit will contain amounts of active ingredients depending on the severity of the cognitive decline disorder and related symptoms, condition, further ongoing therapies, individual state of health and response to the association of active ingredients.

In some embodiments, the dose is in the range of 0.001% by weight to about 60% by weight of the formulation.

According to some embodiments, the composition of the invention is a food supplement in the form of a two-part kit. According to the latter embodiment, a component of the kit is a capsule containing fish oil and a saffron extract containing crocin, and preferably safranal, and the other is a tablet containing NAC and a saffron extract containing crocin and safranal.

According to some embodiments, the use of a kit according to any one of claims 1-7 or a composition according to any one of claims 8-9 is provided, in combination with the simultaneous, separate or subsequent administration of an antidepressant drug, such as for example sertraline.

According to some embodiments, the composition of the invention is a nutraceutical, food supplement, a nutritional product, or a dietetic product.

Typically, daily administration of the composition of the invention improves the symptoms linked to depression and the cognitive abilities after a treatment of at least 4 weeks.

The following examples relate to embodiments and are provided to illustrate the present invention.

EXAMPLE 1

Kit comprising

a) a capsule containing:

fish oil 1000 mg, of which EPA 400 mg, DHA200 mg
dry saffron extract 15 mg, of which crocin 750μg safranal 300 μg;

b) a tablet containing:

N-acetylcysteine 600 mg
dry saffron extract 15 mg, of which crocin 750μg safranal 300 μg;

The tablet has a coating film containing

| Ethylcellulose | 2.2g |
|---|---|
| Talc | 0.2g |
| Carnauba wax | 0.1g |
| Total equal to | 2.5g |

The daily dose for prevention or treatment is 1 or 2 preparations (1 tablet + 1 capsule) per day.

EXAMPLE 2

Composition in the form of a capsule comprising fish oil 1200 mg, of which EPA 600 mg, DHA 300 mg, dry saffron extract 15 mg, of which crocin 750μg, safranal 300 μg, N-acetylcysteine 500 mg.

EXAMPLE 3

Capsule containing crocin 1000 μg, fish oil 1200 mg, of which EPA 600 mg, DHA 300 mg.

EXAMPLE 4

**Cell Viability Test.**

Human microglia cells HMC3 (ATCC® CRL-3304™) were cultured in EMEM (Eagle's minimum essential medium) containing 10% FBS (fetal bovine serum) and PSG (1000 U/ml of Penicillin, 10000 ug/ml of Streptomycin, 29.2 mg/ml of L-Glutamine). The HMC3 cells were placed in 96-well plates with a density of 40,000 cells/well and let to adhere overnight in an incubator at 37°C, 5% CO2. The cells were then treated or not with different concentrations of the substances of the invention (saffron extract (1 μg/mL or 3 μg/mL), NAC (200 μM or 600 μM) or fish oil (8.5 μg/mL or 28.5 μg/mL) dissolved in EMEM with 10% FBS). After 24 hours incubation, cell viability was determined by MTT assay. Then, 10 μL of MTT (5 mg/mL solution) were added to the cells and incubated for 1 hour, followed by removal of the medium and dissolution of the crystals formed in 100 μL of isopropanol. Absorbance at 570 nm was measured with a spectrophotometer (SEAC SIRIO S) and the cell viability was expressed as a percentage of untreated control cells.
As shown in Figure 1, the substances of the invention (saffron extract, NAC or fish oil) do not influence the cell viability of HMC3 cells.

EXAMPLE 5

**ORAC Test (Oxygen Radical Absorbance Capacity).**

The ORAC method was used to assess the antioxidant activity of the substances of the invention by measuring the ability to protect the fluorescent probe (fluorescein) from damage due to peroxyl radical generated by spontaneous decomposition of AAPH (2,2'-azobis (2-amidine-propane) dihydrochloride) at 37°C, with consequent loss of fluorescence. The protective effect of the substances of the invention, alone or in combination, was measured by evaluating the longer area of time/fluorescence intensity under the curve (AUC) of the sample compared to the blank (AUC), wherein substances are not present. Trolox was the antioxidant used as the calibration standard, the ORAC results being expressed as Trolox equivalent micromol/gram of sample (i.e. μmol TE/g).
To test ORAC samples, 0.5 g of dry saffron extract or NAC powder were dissolved in 20 mL of acetone-water mixture (50 + 50, v/v) and mixed by stirring at room temperature. After mixing, the extracts were centrifuged, and the supernatants diluted with 75 mM potassium phosphate buffer (pH 7.4). As for fish oil or the combination (1000 mg of fish oil + 600 mg of NAC + 30 mg of saffron dry extract), 0.5 g of sample were dissolved in 20 mL of acetone, followed by dilution of an aliquot of each solution sample with 7% RMCD (methylated β-cyclodextrin solvent) (w/v) produced in a 50% (v/v) acetone-water mixture. After mixing, the solution samples were diluted with 7% RMCD acetone solvent.
The ORAC test was performed by adding 150 μL of working solution with fluorescein to all the microplate wells, followed by the addition of 25 μL of diluted buffer (white), 50 μL of diluted buffer (control), 25 μL of standard (Trolox 6.25 - 50 μM) or 25 μL of the samples prepared before filling the microplate wells. Then, the microplate was incubated at 37°C for 30 minutes and 25 μL of AAPH were added to all the wells, with the exception of the controls, to obtain the final volume of 200 μL/well. The plate was immediately transferred to the plate reader and the fluorescence was measured every minute for 35 minutes. Fluorescence readings refer to the highest reading of the wells in which no AAPH (controls) is added.

The net AUC of the standards and samples were calculated. The standard curve is obtained by plotting the concentrations of Trolox versus average net AUC of the two measurements for each concentration. The final ORAC values were calculated using the regression equation between Trolox concentration and the net AUC and expressed as Trolox equivalent micromoles/gram of samples. The ORAC value was calculated according to the formula:

$$\text{ORAC Trolox Equivalents μmol/gram} = [(As - Ab) / (At - Ab)] \, kah$$

where As is the area under the curve (AUC) of fluorescein in the sample, At is Trolox AUC Ab is the control AUC, k is the dilution factor, a is the concentration of Trolox in μmol/L and h is the ratio between the liters of extract and the grams of active compounds or oil used for extraction.

As shown in Figure 2, the antioxidant activity of the combination (striped bar) is much higher than that of the single substances with the same percentage composition used in the combination (white bars). In fact, a clear synergistic effect is observed, since the sum of TE μmol/g of the substances alone (98.49 TE μmol/g for fish oil + 24.04 TE μmol/g for NAC + 329.25 for dry saffron extract) is lower than that obtained for the combination (451.78 TE μmol/g compared to 597.7 TE μmol/g for the combination).

<u>EXAMPLE 6</u>

**Modulation of cytokine secretion in HMC3 cells exposed or not to inflammatory stimulus after treatment with the substances of the invention.**

HMC3 cells were seeded in 96-well plates with a density of 40,000 cells/well in EMEM containing 10% FBS and PSG (1000 U/mL of penicillin, 10000 ug/mL of streptomycin, 29.2 mg/mL of L-Glutamine) and let to adhere overnight in an incubator at 37°C, 5% $CO_2$. Then, the cells were treated or not with different concentrations of the substances of the invention (saffron extract, NAC, fish oil or combinations thereof) dissolved in EMEM with 3% FBS and incubated for a 24-hour treatment in the presence or in the absence of an inflammatory stimulating agent (TNFα 50 ng/mL dissolved in EMEM with 3% FBS). At the end of the treatment, the cell-free supernatants were collected and frozen at -80°C until further analysis.

The secretion of human IL8 (hIL8) and human CXCL10 (hCXCL10) cytokines was analyzed by ELISA (Enzyme-Linked Immunosorbent Assay) kits, according to the manufacturer's instructions (BOOSTER BIOLOGICAL TECHNOLOGY Co. LTD) in supernatants free of HMC3 cells treated as described above, that were diluted twice in "sample diluent" supplied in the ELISA kits. The absorbance at 450 nm was measured with a spectrophotometer (SEAC SIRIO S) and fitted with the standard curve and the cytokine concentrations mean was expressed in pg/mL.

Figures 3A and 3B show that both treatment with the substances alone or combinations thereof are sufficient to reduce the release of cytokines in HCM3 cells supernatants, without any inflammatory stimulus. However, the combinations of the substances of the invention show a synergistic effect in reducing the levels of both cytokines, hIL8 and hCXXL10. Furthermore, it is observed that when the HCM3 cells were stimulated with TNFα, the synergistic effect of the combinations in reducing the cytokine levels is even more evident, as can be seen in Figures 4A and 4B. The synergistic effect of the combinations as compared to the reduction of cytokine levels by treatment with the single substances is confirmed by Figures 5A and 5B.

**Claims**

1.    A kit comprising

   a) a composition comprising an omega-3 fatty acid and crocin and a physiologically acceptable carrier, and
   b) a composition comprising N-acetylcysteine and crocin and a physiologically acceptable carrier.

2.    The kit according to claim 1, wherein the composition a) is in the form of a capsule and the composition b) is in the form of a tablet.

**3.** The kit according to claims 1 or 2, wherein the crocin is contained in a saffron extract, preferably a dry saffron extract.

**4.** The kit according to claim 3, wherein the saffron extract further contains safranal.

**5.** The kit according to any one of claims 1 to 4, wherein said omega-3 fatty acid is contained or originates from fish oil.

**6.** The kit according to claim 5, wherein the fish oil is in an amount of 100 to 1500 mg, the saffron extract in an amount of 10 to 50 mg and N-acetylcysteine in an amount of 250 to 750 mg.

**7.** The kit according to any one of claims 1-6, wherein said omega-3 fatty acid comprises or consist of EPA and/or DHA.

**8.** The kit according to any one of claims 1-7, for use as a combined preparation for simultaneous, separate or subsequent administration of compositions a) and b) in the prevention or treatment of depression or a symptom thereof and/or a cognitive disorder linked to depression.

**9.** The kit for use according to claim 8 in the prevention or treatment of a symptom of a depressive disorder or a cognitive disorder linked to a depressive disorder.

**10.** A composition comprising a saffron extract and N-acetylcysteine and/or fish oil and a physiologically acceptable carrier.

**11.** A composition comprising crocin and N-acetylcysteine and/or an omega-3 fatty acid and a physiologically acceptable carrier.

**12.** The composition according to any one of claims 10 or 11 for use in the prevention of a symptom of a depressive disorder or a cognitive disorder linked to a depressive disorder.

**13.** The kit according to any one of claims 1 to 9 or the composition according to any one of claims 10 or 12, **characterized in that** it is a food supplement, a nutraceutical, a medicine or a nutritional product.

**Figure 1**

**Figure 2**

**Figure 3A**

**Figure 4A**

Figure 3B

**Figure 4B**

**Figure 5A**

**Figure 5B**

Europäisches
Patentamt

European
Patent Office

Office européen
des brevets

# EUROPEAN SEARCH REPORT

Application Number

EP 20 15 8390

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 9 833 489 B2 (BOURGES CEDRIC [FR]) 5 December 2017 (2017-12-05) * column 1, line 7 - line 45 * * column 2, line 62 - line 67 * * column 5, line 30 - line 31 * * column 6, line 55 - line 65; table 3 * * column 8, line 26 - line 63 * * claims 1-3, 11, 12 * | 1-13 | INV. A61K9/48 A61K31/202 A61K31/351 A61K36/744 A61K45/06 |
| X | US 2016/361272 A1 (THOMAOGLOU CONSTANT [MC]) 15 December 2016 (2016-12-15) * paragraph [0002] - paragraph [0051] * * paragraph [0078] - paragraph [0109] * * paragraph [0124] * * claims 1-10 * | 1-13 | |
| A | PETER DOME ET AL: "Natural health products, dietary minerals and over-the-counter medications as add-on therapies to antidepressants in the treatment of major depressive disorder: a review", BRAIN RESEARCH BULLETIN., vol. 146, 30 December 2018 (2018-12-30), pages 51-78, XP055637252, ISSN: 0361-9230, DOI: 10.1016/j.brainresbull.2018.12.015 * abstract * * page 52, left-hand column, paragraph 2 - page 54, left-hand column * * 3.1. Omega-3 fatty acids 3.2. Saffron 3.21. N-acetylcysteine * * page 67, right-hand column, last paragraph - page 68, right-hand column * | 1-13 | TECHNICAL FIELDS SEARCHED (IPC) A61K |

The present search report has been drawn up for all claims

| Place of search | Date of completion of the search | Examiner |
|---|---|---|
| The Hague | 15 June 2020 | González Ferreiro, M |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

## ANNEX TO THE EUROPEAN SEARCH REPORT
## ON EUROPEAN PATENT APPLICATION NO.

EP 20 15 8390

This annex lists the patent family members relating to the patent documents cited in the above-mentioned European search report.
The members are as contained in the European Patent Office EDP file on
The European Patent Office is in no way liable for these particulars which are merely given for the purpose of information.

15-06-2020

| Patent document cited in search report | | | Publication date | Patent family member(s) | | Publication date |
|---|---|---|---|---|---|---|
| US 9833489 | B2 | | 05-12-2017 | AU | 2007245501 A1 | 08-11-2007 |
| | | | | CA | 2648985 A1 | 08-11-2007 |
| | | | | DK | 2010012 T3 | 06-03-2017 |
| | | | | EP | 2010012 A1 | 07-01-2009 |
| | | | | ES | 2616128 T3 | 09-06-2017 |
| | | | | FR | 2900053 A1 | 26-10-2007 |
| | | | | HU | E031843 T2 | 28-08-2017 |
| | | | | JP | 6093425 B2 | 08-03-2017 |
| | | | | JP | 2009534452 A | 24-09-2009 |
| | | | | JP | 2014015480 A | 30-01-2014 |
| | | | | JP | 2016053062 A | 14-04-2016 |
| | | | | PL | 2010012 T3 | 31-07-2017 |
| | | | | PT | 2010012 T | 22-02-2017 |
| | | | | US | 2010028464 A1 | 04-02-2010 |
| | | | | US | 2011236481 A1 | 29-09-2011 |
| | | | | US | 2018104299 A1 | 19-04-2018 |
| | | | | WO | 2007125243 A1 | 08-11-2007 |
| US 2016361272 | A1 | | 15-12-2016 | BE | 1023296 A1 | 26-01-2017 |
| | | | | EP | 3110413 A1 | 04-01-2017 |
| | | | | FR | 3017799 A1 | 28-08-2015 |
| | | | | LU | 92643 A2 | 11-07-2016 |
| | | | | US | 2016361272 A1 | 15-12-2016 |
| | | | | WO | 2015124318 A1 | 27-08-2015 |

EPO FORM P0459

For more details about this annex : see Official Journal of the European Patent Office, No. 12/82

**EP 3 698 777 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **TALLARIDA RJ.** Drug Synergism: its detection and applications. *J PharmacolExpTher.,* September 2001, vol. 298 (3), 865-72